(19) Europäisches Patentamt
European Patent Office
Office européen des brevets

(11) Veröffentlichungsnummer: **0 238 925**
**B1**

(12) # EUROPÄISCHE PATENTSCHRIFT

(45) Veröffentlichungstag der Patentschrift: **28.02.90**

(51) Int. Cl.[5]: **C 07 C 271/06**

(21) Anmeldenummer: **87103345.2**

(22) Anmeldetag: **09.03.87**

(54) **Verfahren zur Herstellung N,N-disubstituierter Mono- und Oligourethane.**

(30) Priorität: **22.03.86 DE 3609813**

(43) Veröffentlichungstag der Anmeldung:
**30.09.87 Patentblatt 87/40**

(45) Bekanntmachung des Hinweises auf die Patenterteilung:
**28.02.90 Patentblatt 90/09**

(84) Bennante Vertragsstaaten:
**AT BE CH DE FR GB IT LI NL SE**

(56) Entgegenhaltungen:

**CHEMICAL ABSTRACTS, Band 92, Nr. 7, 18. Februar 1980, Seite 644, Zusammenfassung Nr. 58478c, Columbus, Ohio, US; S. JULIA et al.: "Phase-transfer catalysis. IV. N-alkylation of naphthyl carbamates"**

**CHEMICAL ABSTRACTS, Band 103, Nr. 8, 26. August 1985, Seite 399, Zusammenfassung Nr. 59990w, Columbus, Ohio, US; E.V. DEHMLOW et al.: "Applications of phase-transfer catalysis. Part 30. A comparison of the effectiveness of various aqueous and solid bases and their mixtures in the N-alkylation of benzamide"**

(73) Patentinhaber: **BAYER AG**
**D-5090 Leverkusen 1 Bayerwerk (DE)**

(72) Erfinder: **Sanders, Josef, Dr.**
**Wolfskaul 6**
**D-5000 Köln 80 (DE)**
Erfinder: **Dieterich, Dieter, Dr.**
**H.T. von-Böttinger-Strasse 16**
**D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 238 925 B1

LIBERGRAF, STOCKHOLM 1990

**Beschreibung**

Die Erfindung betrifft ein Verfahren zur Herstellung N,N-disubstituierter Mono- und Oligourethane durch Umsetzung N-aliphatisch, -cycloaliphatisch sowie -araliphatisch mono-substituierter Mono- und Oligourethane mit Alkylierungsmitteln in Gegenwart mindestens äquivalenter Mengen an festem Metallhydroxid in Substanz oder in einem geeigneten Lösungsmittel, gegebenenfalls in Gegenwart eines Phasentransferkatalysators.

Es ist bereits bekannt, daß Monourethane mit niederen Alkylhalogeniden bzw. Alkylsulfaten zu N,N-disubstituierten Monourethanen umgesetzt werden können (vgl. U. Petersen in Houben-Weyl, Bd. E 4, Herausgeber Hagemann). Nachteilig bei den bekannten Verfahren ist jedoch, daß gute Ergebnisse nur dann erzielt werden, wenn dazu spezielle, relativ teure Basen wie Metallhydride, z. B. NaH, eingesetzt werden. Wegen der unter diesen Reaktionsbedingungen dominierenden Nebenreaktion der Olefinbildung beim Einsatz sekundärer Alkylierungsmittel sind diese Verfahren darüber hinaus auf den Einsatz primärer Alkylierungsmittel beschränkt.

Es ist weiterhin bekannt, daß N-arylsubstituierte Monourethane unter den Bedingungen der Phasentransferkatalyse N-alkyliert werden können. Zwar können nach diesem Verfahren auch sekundäre Alkylierungsmittel eingesetzt werden, die Methode versagt jedoch bei N-aliphatischsubstituierten Urethanen völlig (vgl. S. Julia, A. Ginebreda, Anales de Ouimica (Madrid), Vol. 75, S. 348, Zeile 7 - 13).

In den dort beschriebenen Beispielen wird als Lösungsmittel entweder Methylenchlorid, Dimethylsulfoxid oder Methylethylketon verwendet und in jedem Fall Triethylbenzylammoniumchlorid als Phasentransferkatalysator zugesetzt. Diese Lösungsmittel zeigen jedoch Nachteile, wodurch die Ausbeuten der Reaktion zum Teil beträchtlich vermindert werden. Beispielsweise wirkt Methylenchlorid unter diesen Reaktionsbedingungen selbst als Alkylierungsmittel, während Methylethylketon durch Selbstkondensation aldolartige Nebenprodukte bildet. Dimethylsulfoxid bildet giftige, übelriechende Nebenprodukte und ist zudem aus den Reaktionsprodukten nur schwer zu entfernen.

Außerdem erschweren hier die für die Umstezungen notwendigen Phasentransferkatalysatoren in vielen Fällen die Aufarbeitung der Reaktionsmischungen durch Emulsionsbildung; sie sind zudem aus den Reaktionsprodukten oft nur sehr mühsam vollständig zu entfernen.

Aufgabe dieser Erfindung war es, ein wirtschaftliches Verfahren zur Alkylierung N-monosubstituierter Urethane zu entwickeln, das die beschriebenen Nachteile vermeidet. Es wurde nun überraschenderweise gefunden, daß man die gewünschten N,N-disubstituierten Mono- und Oligourethane erhält, wenn man N-aliphatisch, -cycloaliphatisch oder -araliphatisch monosubstituierte Mono- und Oligourethane mit Alkylierungsmitteln in Gegenwart mindestens äquivalenter Mengen an festem Metallhydroxid gegebenenfalls in Gegenwart eines aprotischen, organischen Lösungsmittels und gegebenenfalls in Gegenwart eines Phasentransferkatalysators umsetzt.

Gegenstand der Erfindung ist daher ein Verfahren zur Herstellung von N,N-disubstituierten Mono- oder Obligourethanen, das dadurch gekennzeichnet ist, daß man Nmonosubstituierte Mono- oder Obligourethane der allgemeinen Formel I und II

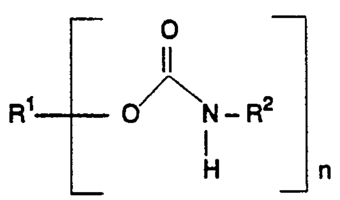

und

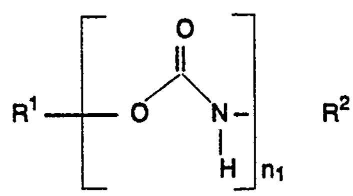

I II

worin

n = eine ganze Zahl von 1 bis 6, vorzugsweise 1 - 4
$n_1$ = eine ganze Zahl von 1 bis 6, vorzugsweise 1 - 3

$R^1$ einen aromatischen Kohlenwasserstoffrest mit vorzugsweise 6 - 18, besonders bevorzugt 6 - 13 C-Atomen, einen aliphatischen Kohlenwasserstoffrest mit vorzugsweise 1 - 18, besonders bevorzugt 1 - 6 C-Atomen, einen cycloaliphatischen Kohlenwasserstoffrest mit vorzugsweise 4 - 30, besonders bevorzugt 6 - 15 C-Atomen oder einen araliphatischen Kohlenwasserstoffrest mit vorzugsweise 7 - 30, besonders bevorzugt 7 - 15 C-Atomen
$R^2$ einen aliphatischen Kohlenwasserstoffrest mit vorzugsweise 1 - 18, besonders bevorzugt 2 - 8 C-Atomen, einen cycloaliphatischen Kohlenwasserstoffrest mit vorzugsweise 4 - 30, besonders bevorzugt 6 - 15 C-Atomen oder einen araliphatischen Kohlenwasserstoffrest mit vorzugsweise 7 - 20, besonders bevorzugt 7 - 13 C-Atomen bedeutet,

mit einem Alkylierungsmittel der allgemeinen Formel III

$$R^3X \qquad \text{III}$$

in der

$R^3$ einen aromatischen Kohlenwasserstoffrest mit vorzugsweise 6 - 18, besonders bevorzugt 6 - 13 C-Atomen, einen aliphatischen Kohlenwasserstoffrest mit vorzugsweise 1 - 18, besonders bevorzugt 1 - 12 C-Atomen, einen cycloaliphatischen Kohlenwasserstoffrest mit vorzugsweise 7 - 30, besonders bevorzugt 7 - 15 C-Atomen oder einen araliphatischen Kohlenwasserstoffrest mit vorzugsweise 7 - 39, besonders bevorzugt 7 - 15 C-Atomen bedeutet und

X für ein Halogenatom, vorzugsweise Cl, Br, eine Sulfat-, Sulfonat-, Phosphat- oder Phosphonatgruppe steht,

in Gegenwart mindestens äquivalenter Mengen eines festen Metallhydroxides, gegebenenfalls in Gegenwart eines aprotischen organischen Lösungsmittels und gegebenenfalls in Gegenwart eines Phasentransferkatalysators umsetzt.

Die erfindungsgemäß erhaltenen N,N-disubstituierte Mono- und Oligourethane entsprechen den allgemeinen Formeln IV und V

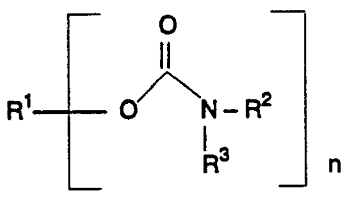

und

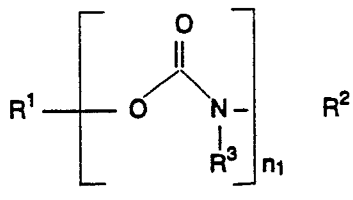

IV

V

in der n und $n_1$, $R^1$, $R^2$ und $R^3$ die oben angegebene Bedeutung haben.

Zur Erzielung guter Ausbeuten ist es besonders vorteilhaft, das erfindungsgemäße Verfahren derart durchzuführen, daß man das Ausgangsurethan, das Alkylierungsmittel und gegebenenfalls den Phasentransferkatalysator in einem aprotischen organischen Lösungsmittel - bevorzugt werden Chlorbenzol, Dimethylformamid und N-Methylpyrrolidonoder in überschüssigem Alkylierungsmittel - löst, dann das Metallhydroxid (bevorzugt Natrium- oder Kaliumhydroxid) in fester Form, portionsweise oder kontinuierlich bei niedrigen Reaktionstemperaturen, z. B. 20 - 30°C gegebenenfalls unter Kühlung, zugibt und anschließend bis zum vollständigen Umsatz, gegebenenfalls unter Erwärmen auf 50 - 80°C, nachrührt. Auf den Zusatz eines Phasentransferkatalysators kann bei Verwendung von polaren aprotischen Lösungsmitteln wie z. B. Dimethylformamid, N-Methylpyrrolidon oder Dimethylsulfoxid verzichtet werden, wodurch sich die Aufarbeitung der Reaktionsgemische wesentlich vereinfacht.

Im Vergleich zu den bekannten Verfahren liefert das erfindungsgemäße Verfahlen überraschend auf einfacherem und wirtschaftlicherem Wege N,N-disubstituierte Urethane in besserer Ausbeute, besserer Raum-Zeit-Ausbeute und Reinheit und im technischen Maßstab.

So kommen hier im Gegensatz zu den bekannten Verfahren statt teurer und gefährlicher Metallhydride preisgünstigere, ungefährlicher Metallhydride preisgünstigere, ungefährlichere und einfacher handhabbare Metallhydroxide zum Einsatz.

Es muß ganz besonders überraschen, daß nach dem erfindungsgemäßen Verfahren im Gegensatz zur Literatur auch N-monoaliphatisch substituierte Urethane alkyliert werden können.

Als Ausgangsmaterial für das erfindungsgemäße Verfahren kommen Urethane in Betracht, die sich z. B. durch Umsetzung von vorzugsweise aliphatischen Mono- und Oligoisocyanaten mit ein- bis sechswertigen Alkoholen nach bekannten Verfahren in der Schmelze oder in Lösung, gegebenenfalls in Gegenwart eines Katalysators, herstellen lassen.

Diese Urethane können über beispielsweise auch durch Kondensation primärer Mono- oder Oligoamine mit Chlorameisensäureestern ein- bis sechswertiger Alkohole hergestellt werden. Selbstverständlich kann man zu ihrer Herstellung auch Carbamidsäurechloride mit Alkoholen umsetzen.

Zur Herstellung der Ausgangsurethane für das erfindungsgemäße Verfahren können beispelsweise eingesetzt werden:

Alkohole der Formel

$R^1(OH)_n$

in der $R^1$ und n die oben angegebene Bedeutung haben.

Hier kommen beispielsweise einwertige Alkohole in Frage, wie sie in Ullmanns Enzyklopädie der Technischen Chemie, Bd. 7, S. 205 - 206, 4. Auflage, 1974 beschrieben sind, außerdem Phenole sowie substiutierte Phenole.

Als mehrwertige Alkohole kommen z. B. Ethylenglykol. Propylenglykol-(1,2) und -(1,3), Butylenglykol-(1,4) und -(2,3), Hexandiol-(1,6), Octandiol-(1,8), Neopentylglykol, 1,4-Bis-hydroxymethylcyclohexan, 2-Methyl-1,3-propandiol, Glycerin, Trimethylolpropan, Hexantriol-(1,2,6), Butantriol-(1,2,4), Trimethylolethan, Pentaerythrit, Chinit, Mannit und Sorbit, Formit, Methylglykosid, und/oder 1,4-, 3,6-Dianhydrohexite, außerdem mehrwertige Phenole wie z. B. Brenzcatechin, Resorcin, Hydrochinon sowie mehrkernige Phenole z. B. vom Typ des Bisphenol A in Frage.

Selbstverständlich können auch Mischungen dieser Alkohole eingesetzt werden.

Isocyanate zur Herstellung der Ausgangsprodukte entsprechen die allgemine Formel $R^2(NCO)_n$, in der $n_1$ und $R^2$ die oben angegebene Bedeutung haben.

Beispielsweise können eingesetzt werden: Isocyanatomethan, -ethan, -propan, -butan, -pentan, -hexan, 6-Chlorhexylisocyanat, Isocyanatocyclohexan, Benzylisocyanat, Tetramethylendiisocyanat; Hexamethylendiisocyanat, Dekamethylendiisocyanat; 1,3-Di-(3-isocyanatopropoxy)-2,2-dimethylpropan; Cyclohexandiisocyanat-(1,4); Methylcyclohexandiisocyanat-(2,4); Methylcyclohexandiisocyanat-(2,6); 1,3-Diisocyanatocyclohexan, Gemische aus Methylcyclohexandiisocyanat-(2,4) und Methylcyclohexandiisocyanat-(2,6); Dicyclohexylmethan-4,4 -diisocyanat; 1-Isocyanato-3-isocyanatomethyl-3,5,5-trimethylcyclohexan (Isophorondiisocyanat); 1,2-Di-(isocyanatomethyl)-cyclobutan; m- und p-Xylylendiisocyanat sowie α,α,α',α'-Tetramethyl-m-und/oder -p-xylylendiisocyanat oder -hexahydroxylylendiisocyanat. Bei cycloaliphatischen Diisocyanaten können beliebige Stereoisomere oder ihre Gemische Verwendung finden.

Selbstverständlich können auch Mischungen dieser Isocyanate eingesetzt werden.

Als Alkylierungsmittel werden solche der Formel

$R^3X$

eingesetzt, in der $R^3$ und X die oben angegebene Bedeutung haben.

Selbstverständlich kann der Kohlenwasserstoff $R^3$ neben der Abgangsgruppe X durch weitere funktionelle Gruppen substituiert sein, wenn diese unter den erfindungsgemäßen Reaktionsbedingungen inert sind oder in definierter Weise mit den erfindungsgemäßen Reagenzien reagieren, z. B. Nitro-, bestimmte Ester-, Urethan-Amidgruppen und Sulfonylgruppen, nicht aktiviertes, aromatisch gebundenes Halogen, Epoxidgruppen, Aziridingruppen, Ethergruppen, Thioethergruppen und dergleichen mehr.

Als Alkylierungsmittel kommen beispielsweise in Frage:

Methylchlorid, -bromid, Ethyl-chlorid, -bromid, Propylchlorid, -bromid, i-Propyl-chlorid, -bromid, n-Butylchlorid, -bromid, Iso-butyl-chlorid, -bromid, Cyclohexyl-chlorid, -bromid, Octyl-, Nonyl-, Decyl-, Undecyl-, Dodecyl-chlorid und -bromid, Benzyl-chlorid, -bromid, Allyl-chlorid, -bromid, p-Nitrobenzyl-chlorid, -bromid, 2,4-Dinitrochlorbenzol, 2,4-Dinitrofluorbenzol, 2,4,6-Dinitrochlorbenzol, 2,4,6-Dinitrofluorbenzol, Dimethylsulfat, Diethylsulfat, p-Toluolsulfonsäuremethylester, -ethylester, Ethylenchlorhydrin, Ethylenbromhydrin, Epichlorhydrin.

Selbstverständlich können auch Mischungen dieser Alkylierungsmittel eingesetzt werden.

Besonders bevorzugte Alkylierungsmittel sind: Methylchlorid, -bromid, Ethylchlorid, -bromid, Dodecylchlorid, -bromid und Allylchlorid, -bromid, Benzylchlorid, sowie p-Tosylester.

Bei Einsatz leicht flüchtiger Alkylierungsmittel wie z. B. Methylenchlorid, -bromid oder Ethylchlorid ist es zweckmäßig, die Umsetzung in einem Autoklaven unter Druck durchzuführen.

Dabei kann entweder in einem aprotischen organischen Lösungsmittel oder in überschüssigem, verflüssigtem Alkylierungsmittel, gegebenenfalls in Gegenwart eines Phasentransferkatalysators, gearbeitet werden.

Erfindungsgemäß werden feste, vorzugsweise fein pulverisierte Metallhydroxide eingesetzt, vorzugsweise Alkalimetallhydroxide wie Kalium- und Natriumhydroxid. Aus wirtschaftlichen Gründen wird Natriumhydroxid bevorzugt. Selbstverständlich kann man z. B. auch Lithium-, Rubidiumund Bariumhydroxid oder auch feuchtes Silberoxid einsetzen. Es kann in manchen Fällen vorteilhaft sein, Mischungen dieser Metallhydroxide einzusetzen. Mindestens äquivalente Mengen, bezogen auf Urethangruppen, werden eingesetzt.

Die oben beschriebenen Ausgangsurethane können mit dem Alkylierungsmittel in (bezogen auf die Anzahl im Molekül vorhandener Urethangruppen) stöchiometrischer Menge, im Überschuß oder Unterschuß umgesetzt werden. Bevorzugt wird ein Verhältnis von 0,3 - 5 Mol, ganz besonders bevorzugt 12 Mol Alkylierungsmittel pro Mol Urethangruppen. Selbstverständlich wird bei Verwendung eines Unterschusses an Alkylierungsmittel nur eine Teilalkylierung erreicht werden, während die Verwendung eines größeren Überschusses an Alkylierungsmittel unwirtschaftlich ist.

Die Umsetzung wird im allgeminen bei einer Temperatur von 0 - 180°C, bevorzugt bei 10 - 80°C, ganz besonders bevorzugt bei Raumtemperatur mit Überdruck, Unterdruck oder zweckmäßig drucklos, kontinuerlich oder diskontinuierlich durchgeführt.

Die Verweilzeit beträgt vorzugsweise 0,5 - 24 Stunden, besonders bevorzugt 0,5 - 10 Stunden.

Zweckmäßig führt man die Reaktion in überschüssigem Alkylierungsmittel oder bevorzugt in einem organischen Lösungsmittel durch.

Geeignet sind solche aprotischen organischen Lösungsmittel, die unter den erfindungsgemäßen Reaktionsbedingungen inert sind und z. B. in Ullmanns Enzyklopädie der Technischen Chemie Bd. 14, 4. Auflage, Verlag Chemie 1978, S. 305 beschrieben sind. Als Lösungsmittel kommen z. B. in Frage: Benzol, Toluol, Xylol, Ethylbenzyl, Cumol, Methylenchlorid, Chloroform, Dichorbenzol, Trichlorbenzol, Nitrobenzol, Aceton, Methylethylketon, Diethylketon, Cyclohexanon, Diethylether, Diisopropylether, Tetrahydrofuran, Dioxan, Dimethylformamid, Dimethylacetamid, Dimethylsulfoxid, Tetramethylensulfon, Furfurol, Nitromethan, Nitroethan, Nitropropan, N-Methylpyrrolidon, Hexamethylenphosphonsäuretriamid.

Bevorzugt werden Chlorbenzol, Dimethylformamid, NMethylpyrrolidon und Tetramethylensulfon.

Selbstverständlich können auch Mischungen dieser Lösungsmittel verwendet werden.

Es kann in manchen Fällen vorteilhaft sein, die Umsetzung in Gegenwart eines Phasentransferkatalysators durchzuführen. Solche Katalysatoren werden z. B. in E.V. und S.S. Dehmlow, Phase Transfer Catalysis, 2. Auflage, Verlag Chemie 1983, beschrieben. Gegeignete Katalysatoren sind quaternäre Ammonium- oder Phosphoniumsalze der Formel VI

$$\left[\begin{array}{c} R'' \\ | \\ R' - Z - R''' \\ | \\ R'''' \end{array}\right]^{(+)} \quad A^{(-)} \qquad VI$$

in welcher

Z für Stickstoff oder Phosphor steht,
R' ,R", R"' und R"" gleich oder verschieden, eine Alkylgruppe mit 1 bis 18 Kohlenstoffatomen bedeutet, wobei einer der Reste auch für einen araliphatischen Rest mit 7 bis 15 Kohlenstoffatomen stehen kann, und wobei die Summe der Kohlenstoffatome der vier Reste R' - R" vorzugsweise 12 bis 29 beträgt, und $A^-$ ein Anion, vorzugsweise ein Halogenid oder Phosphonat, bedeutet.

Typische Beispiele geeigneter Katalysatoren sind N-Benzyl-N,N,N-triethyl-ammoniumchlorid oder -bromid, N-Benzyl-Ndodecyl-N,N-dimethyl-ammoniumchlorid oder -bromid, N,N,N,N-Tetra-n-hexyl-ammoniumchlorid oder -bromid, N-Benzyl-N,N,N-tri-n-octyl-ammoniumchlorid oder -bromid oder die diesen Ammoniumsalzen entsprechenden Phosphoniumsalze.

Die beispielhaft genannten quarternären Ammonium- oder Phosphoniumsalze werden bei der Durchführung des erfindungsgemäßen Verfahrens vorzugsweise in Substanz oder in Form ihrer wäßrigen Lösungen (beispielsweise mit einem Feststoffgehalt von 30 bis 60 Gew.-%) und vorzugsweise in einer Menge von 1 bis 10 Mol-%, bezogen auf die Molzahl der vorhandenen Urethangruppen eingesetzt.

Bei Verwendung von polaren aprotischen Lösungsmitteln wie z. B. Dimethylformamid, N-Methylpyrrolidon, Dimethylsulfoxid und Sulfolan kann ohne Nachteile auf den Zusatz von Phasentransferkatalysatoren verzichtet werden.

Zur Durchführung des erfindungsgemäßen Verfahrens kann man beispielsweise so vorgehen, daß man das Urethan, das Alkylierungsmittel und gegebenenfalls den Katalysator im gewählten Lösungsmittel vorlegt und das feste, möglichst feingemahlene Metallhydroxid unter Rühren gegebenenfalls unter Kühlung portionsweise oder kontiuierlich zugibt. Anschließend rührt man bei Raumtemperatur oder gegebenenfalls bei erhöhter Temperatur so lange nach, bis die dünnschichtchromatographische oder gaschromatographische Analyse vollständigen Umsatz anzeigt.

Die Aufarbeitung kann nach an sich bekannten Verfahren erfolgen. Bei Verwendung von mit Wasser misch~~baren Lösungsmittel kann man im Fall fester, in Wasser unlöslicher Reaktionsprodukte das Reaktionsgemisch in~~ Wasser einrühren und das ausgefallene Reaktionsprodukt in üblicher Weise durch Absaugen isolieren. Im Fall öliger Reaktionsprodukte ist dagegen eine extraktive Aufarbeitung nach üblichen Methoden sweckmäßig. Die Rohprodukte können kristallisation oder Destillation, gereinigt werden.

Die Aufarbeitung kann nach an sich bekannten Verfahren erfolgen. Bei Verwendung von mit Wasser mischbaren Lösungsmittel kann man im Fall fester, in Wasser unlöslicher Reaktionsprodukte das Reaktionsgemisch in Wasser einrühren und das ausgefallene Reaktionsprodukt in üblicher Weise durch Absaugen isolieren. Im Fall öliger Reaktionsprodukte ist dagegen eine extraktive Aufarbeitung nach üblichen Methoden zweckmäßig. Die Rohprodukte können falls erforderlich-, in üblicher Weise, z. B. durch Umkristallisation oder Destillation, gereinigt werden.

Die nach dem Verfahren der Erfindung herstellbaren N,N-disubstituierten Urethane sind Wirkstoffe und wertvolle Ausgangsstoffe für die Herstellung von Farbstoffen, Pharmazeutika und thermostabilen Kunststoffen. Insbesondere zeigen die erfindungsgemäßen N,N-disubstituierten Urethane größere thermische, thermooxidative und photooxidative Stabilität (vgl. R. Vieweg, A. Höchtlen, Kunststoff Handbuch Bd. VII, Polyurethane, Hauser Verlag München 1966, S. 11, S. 21), sowie ein günstigeres Brandverhalten als die entsprechende N-monosubstituierten Urethane.

Durch Hydrolyse der N,N-disubstituierten Urethane können die entsprechend substituierten sekundären Amine hergestellt werden, die ebenfalls wichtige Ausgangsprodukte für die Synthese von Wirkstoffen sowie die Herstellung und Formulierung von Kunststoffen darstellen.

Alle Reaktionsprodukte wurden gaschromatographisch oder dünnschichtchromatographisch auf ihre Reinheit überprüft und ihre Identität durch Aufnahme von IR- und NMR-Spektrum gesichert.

Gerade die IR-Spektroskopie gestattet eine bequeme Umsatzkontrolle: Im Verlauf der Reaktion verschwinden die für N-monosubstituierte Urethane charakteristischen Banden bei 3200 - 3500 $cm^{-1}$ (ν N-H) und 1530 - 1560 $cm^{-1}$ (δ N-H).

**Beispiel**

**Beispiel 1**

Zu einer Lösung von 58,1 g N-6-Chlorhexylcarbamidsäuremethylester und 51,7 g Diethylsulfat in 300 ml Dimethylformamid (DMF) gibt man unter Rühren bei 20°C 16 g gepulvertes Natriumhydroxid im Verlauf von zwei Stunden portionsweise zu. Nach Zugabe rührt man noch weitere drei Stunden bei Raumtemperatur, zieht das Lösungsmittel im Vakuum ab und nimmt den Rücksland in 700 ml Methylenchlorid auf. Die organische Phase wird erst mit gesättiger $NH_4Cl$-Lösung, dann mit Wasser gewaschen und über $Na_2SO_4$ getrocknet. Nach Abziehen des Lösungsmittels wird das ölige Rohprodukt im Hochvakuum fraktioniert.

Ausbeute: 47,8 g (72 %, Kp: 90°C/0,13 mbar (farbloses Öl).

**Beispiel 2**

52,4 g N-tert.-Butylcarbamidsäuremethylester, 57 g Benzylchlorid und 18 g gepulvertes Natriumhydroxid werden analog Beispiel 1 in 300 ml DMF umgesetzt.

Nachrührzeit: 15 Stunden (h) bei 50°C
Ausbeute: 43,3 g (49 %), Kp: 90°C/0,13 mbar (farbloses Öl)

**Beispiel 3**

49,5 g N-Benzylcarbamidsäuremethylester, 25,2 g Allylchlorid und 13,2 g gepulvertes Natriumhydroxid werden analog Beispiel 1 in 300 ml DMF umgesetzt.

Nachrührzeit: 15 h bei 25°C
Ausbeute: 51,l g (83 %), Kp: 118°C/0,16 mbar (farbloses Öl)

**Beispiel 4**

49,5 g N-Benzylcarbamidsäuremethylester, 48 g n-Butylbromid und 14 g gepulvertes Natriumhydroxid werden analog Beispiel 1 in 300 ml DMF umgesetzt.

Nachrührzeit; 15 h bei 50°C
Ausbeute: 50,4 g (76 %), Kp: 110°C/0,2 mbar (farbloses Öl)

**Beispiel 5**

49,5 g N-Benzylcarbamidsäuremethylester, 48 g n-Butylbromid, 12,1 g Methyl-tridecylammoniumchlorid und 14 g gepulvertes Natriumhydroxid werden analog Beispiel 1 in 400 ml Chlorbenzol umgesetzt.

Nachrührzeit: 15 h bei 50°C
Ausbeute: 45,1 g (68 %), Kp: 110°C/0,2 mbar (farbloses Öl)

**Beispiel 6**

49,5 g N-Benzylcarbamidsäuremethylester, 61,4 g p-Toluolsulfonsäuremethylester und 13,2 g gepulvertes Natriumhydroxid werden analog Beispiel 1 in 300 ml DMF umgesetzt.

Nachrührzeit: 15 h bei 25°C
Ausbeute: 48,9 g (92 %), Kp: 96°C/0,44 mbar (farbloses Öl)

**Beispiel 7**

49,5 g N-Benzylcarbamidsäuremethylester, 49,2 g 2-Brompropan und 16 g gepulvertes Natriumhydroxid werden analog Beispiel 1 in 400 ml DMF umgesetzt.

Nachrührzeit: 15 h bei 25°C
Ausbeute: 19,9 g (32 %), Kp: 89°C/0,45 mbar (farbloses Öl)

**Beispiel 8**

49,5 g N-Benzylcarbamidsäuremethylester, 67,5 g Dodecylchlorid und 24 g gepulvertes Natriumhydroxid werden analog Beispiel 1 in 400 ml DMF umgesetzt.

Nachrührzeit: 15 h bei 50°C
Ausbeute: 85 g (85 %) (gelbliches Öl)

**Beispiel 9**

49,5 g N-Benzylcarbamidsäuremethylester, 30,5 g Epichlorhydrin, 9,7 g Tetrabutylammoniumbromid und 18,5 g gepulvertes Kaliumhydroxid werden analog Beispiel 1 in 400 ml Chlorbenzol umgesetzt.

Nachrührzeit: 15 h bei 25°C
Ausbeute: 37,8 g (57 %), Kp: 135°C/0,53 mbar (farbloses Öl)

**Beispiel 10**

46,4 g Diurethan (hergestellt aus Hexamethylendiisocyanat und Methanol), 81,8 g p-Toluolsulfonsäuremethylester und 17,6 g gepulvertes Natriumhydroxid werden analog Beispiel 1 in 400 ml N-Methyl-pyrrolidon (NMP) umgesetzt.

Nachrührzeit: 15 h bei 50°C
Ausbeute: 42,6 g (82 %) (farbloses Öl)

**Beispiel 11**

46,4 g Diurethan (hergestellt aus Hexamethylendiisocyanat und Methanol), 942 g Allylchlorid und 22 g gepulvertes Natriumhydroxid werden analog Beispiel 1 in 300 ml DMF umgesetzt.

Nachrührzeit: 15 h bei 50°C
Ausbeute: 55,5 g (89 %), Kp: 159 °C/0,11 mbr (farbloses Öl)

**Beispiel 12**

57,2 g Diurethan (hergestellt aus Isophorondiisocyanat und Methanol), 9101,3 g Benzylchlorid und 32 g gepulvertes Natriumhydroxid werden analog Beispiel 1 in 500 ml Tetramethylensulfon (Sulfolan®) umgesetzt.

Nachrührzeit: 15 h bei 60°C
Ausbeute: 71 g (76 %) (gelbliches Öl)

**Beispiel 13**

57,5 g Diurethan (hergestellt aus 1,4-Cyclohexandiisocyanat und Methanol) 42,1 g Allylchlorid und 22 g gepulvertes Natriumhydroxid werden analog Beispiel 1 in 500 ml N-Methylpyrrolidon umgesetzt.

Nachrührzeit: 15 h bei 50°C
Ausbeute: 60,5 g (78 %) (gelbliches Öl)

**Beispiel 14**

65,6 g Diurethan (hergestellt aus Benzylisocyanat und Ethylenglykol) 63,3 g Benzylchlorid und 20 g gepulvertes Natriumhydroxid werden analog Beispiel 1 in 500 ml DMF umgesetzt.

Nachrührzeit: 15 h bei 50°C
Ausbeute: 95,5 g (94 %), Fp: 102°C (farblose Kristalle aus Isopropanol)

**Beispiel 15**

53,3 g Triurethan (hergestellt aus Benzylisocyanat und Trimethylolpropan) 25,3 g Allylchlorid und 18,5 g gepulvertes Kaliumhydroxid werden analog Beispiel 1 in 300 ml Tetramethylensulfon umgesetzt.

Nachrührzeit: 15 h bei 50°C
Ausbeute: 50,9 g (78 %) (gelbliches Öl)

**Beispiel 16**

49,5 g N-Benzylcarbamidsäuremethylester, 66,8 g 2,4-Dinitrochlorbenzol und 13,2 g gepulvertes Natriumhydroxid werden analog Beispiel 1 in 400 ml DMF umgesetzt.

Nachrührzeit: 15 h bei 50°C
Ausbeute: 37,8 g (38 %) (violettes Harz)

**Beispiel 17**

49,5 g N-Benzylcarbamidsäuremethylester, 12,8 g gepulvertes Natriumhydroxid und 6,8 g Triethylbenzylammoniumchlorid werden analog Beispiel 1 in 200 ml Benzylchlorid umgesetzt.

Nachrührzeit: 3 h bei 50°C
Ausbeute: 64,3 g (84 %), Kp: 139°C/0,16 mbar (farbloses Öl)

**Beispiel 18**

33 g N-Benzylcarbamidsäuremethylester, 8,8 g gepulvertes Natriumhydroxid und 4,5 g Triethylbenzylammoniumchlorid werden analog Beispiel 1 in 100 ml n-Butylbromid umgesetzt.

Nachrührzeit: 3 h bei 80°C
Ausbeute: 26,1 g (59 %), Kp: 110°C/0,2 mbar (farbloses Öl)

**Beispiel 19**

82,5 g N-Benzylcarbamidsäuremethylester, 70 g Benzylchlorid, 21 g gepulvertes Nariumhydroxid und 11,3 g Triethylbenzylammoniumchlorid werden analog Beispiel 1 in Substanz umgesetzt.

Ausbeute: 68,9 g (54 %), Kp: 139°C/0,16 mbar (farbloses Öl)

**Patentansprüche**

1. Verfahren zur Herstellung N,N-disubstituierter Monound Oligourethane, dadurch gekennzeichnet, daß man N-monosubstituierte Mono- oder Oligourethane der allgemeinen Formel I und II

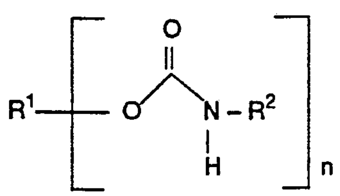

I

und

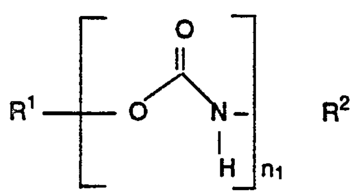

II

worin

n = eine ganze Zahl von 1 bis 6, vorzugsweise 1 - 4
$n_1$ = eine ganze Zahl von 1 bis 6, vorzugsweise 1 - 3
$R_1$ einen aromatischen Kohlenwasserstoffrest mit vorzugsweise 6 - 18 C-Atomen, einen aliphatischen Kohlenwasserstoffrest mit vorzugsweise 1 - 18 C-Atomen, einen cycloaliphatischen Kohlenwasserstoffrest mit vorzugsweise 4 - 30 C-Atomen oder einen araliphatischen Kohlenwasserstoffrest mit vorzugsweise 7 - 30 C-Atomen,

$R_2$ einen aliphatischen Kohlenwasserstoffrest mit vorzugsweise 1 - 18 C-Atomen, einen cycloaliphatischen Kohlenwasserstoffrest mit vorzugsweise 4 - 30 C-Atomen oder einen araliphatischen Kohlenwasserstoffrest mit vorzugsweise 7 - 20 C-Atomen bedeutet, mit einem Alkylierungsmittel der allgemeinen Formel III

$$R^3X \qquad III$$

in der

$R^3$ einen aromatischen Kohlenwasserstoffrest mit vorzugsweise 6 - 18 C-Atomen,
einen aliphatischen Kohlenwasserstoffrest mit vorzugsweise 1 - 18 C-Atomen,
einen cycloaliphatischen Kohlenwasserstoffrest mit vorzugsweise 7 - 30 C-Atomen oder
einen araliphatischen Kohlenwasserstoffrest mit vorzugsweise 7 - 39 C-Atomen bedeutet und
X für ein Halogenatom, vorzugsweise Cl, Br, eine Sulfat-, Sulfonat-, Phosphat- oder Phosphonatgruppe steht,
in Gegenwart mindestens äquivalenter Mengen eines festen Metallhydroxides, gegebenenfalls in Gegenwart eines aprotischen organischen Lösungsmittels und gegebenenfalls in Gegenwart eines Phasentransferkatalysators umsetzt.

2. Verfahren nach Anspruch 1, dadurch gekennzeichnet, daß die Umsetzung ohne Lösungsmittel in überschüssigem Alkylierungsmittel durchgeführt wird.

3. Verfahren nach Anspruch 1, dadurch gekennzeichnet, daß die Umsetzung in einem unpolaren, aprotischen organischen Lösungsmittel gegebenenfalls in Gegenwart von Phasentransferkatalysatoren durchgeführt wird.

4. Verfahren nach Anspruch 3, dadurch gekennzeichnet, daß die Umsetzung in Chlorbenzol, Dimethylformamid, N-Methylpyrrolidon und/oder Tetramethylensulfon durchgeführt wird.

5. Verfahren nach Ansprüchen 1 bis 3, dadurch gekennzeichnet, daß die Umsetzung in Gegenwart von Alkalimetallhydroxiden, vorzugsweise Natriumhydroxid durchgeführt wird.

6. Verfahren nach Ansprüchen 1 bis 4, dadurch gekennzeichnet, daß als Alkylierungsmittel Methylchlorid, -bromid, Ethylchlorid, -bromid, Dodecylchlorid, -bromid und Allylchlorid, -bromid, Benzylchlorid oder p-Tosylester eingesetzt werden.

## Claims

1. A process for the preparation of N,N-disubstituted mono- and oligourethanes, characterised in that N-monosubstituted mono- or oligo-urethanes corresponding to the general formulae I and II

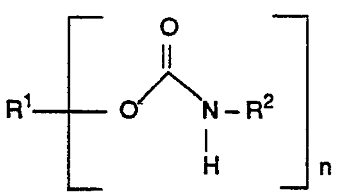

und

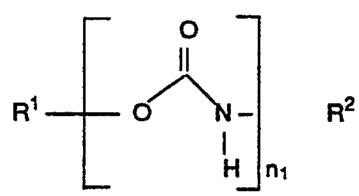

I

II

wherein

n = an integer from 1 - 6, preferably 1 - 4,
$n_1$ = an integer from 1 - 6, preferably 1 - 3,
$R^1$ denotes an aromatic hydrocarbon group preferably containing 6 - 18 carbon atoms, an aliphatic hydrocarbon group preferably containing 1 - 18 carbon atoms, a cycloaliphatic hydrocarbon group preferably containing 4 - 30 carbon atoms or an araliphatic hydrocarbon group preferably containing 7 - 30 carbon atoms,
$R^2$ denotes an aliphatic hydrocarbon group preferably containing 1 - 18 carbon atoms, a cycloaliphatic hydrocarbon group preferably containing 4 - 30 carbon atoms or an araliphatic hydrocarbon group preferably containing 7 - 20 carbon atoms are reacted with an alkylating agent corresponding to the general formula III

$$R^3X \qquad III$$

wherein

$R^3$ denotes an aromatic hydrocarbon group preferably containing 6 - 18 carbon atoms, an aliphatic hydrocarbon group preferably containing 1 - 18 carbon atoms, a cycloaliphatic hydrocarbon group preferably containing 7 - 30 carbon

atoms or an aliphatic hydrocarbon group preferably containing 7 - 39 carbon atoms and

X denotes a halogen atom, preferably Cl or Br, or a sulphate, sulphonate, phosphate or phosphonate group in the presence of at least equivalent quantities of a solid metal hydroxide, optionally in the presence of an aprotic organic solvent and optionally in the presence of a phase transfer catalyst.

2. A process according to claim 1, characterised in that the reaction is carried out solvent-free in excess alkylating agent.

3. A process according to claim 1, characterised in that the reaction is carried out in an apolar aprotic organic solvent, optionally in the presence of phase transfer catalysts.

4. A process according to claim 3, characterised in that the reaction is carried out in chlorobenzene, dimethylformamide, N-methylpyrrolidone and/or tetramethylene sulphone.

5. A process according to claims 1 to 3, characterised in that the reaction is carried out in the presence of alkali metal hydroxides, preferably sodium hydroxide.

6. A process according to claims 1 to 4, characterised in that the alkylating agent used is methyl chloride or bromide, ethyl chloride or bromide, dodecyl chloride or bromide, allyl chloride or bromide, benzyl chloride or p-tosyl ester.

**Revendications**

1. Procédé pour préparer des mono-uréthannes et des oligouréthannes, N,N-disubstitués, procédé caractérisé en ce qu'on fait réagir, en présence de quantités au moins équivalentes d'un hydroxyde de métal solide, éventuellement en présence d'un solvant organique aprotique et éventuellement en présence d'un catalyseur de transfert de phase des mono-uréthannes ou des oligo-uréthannes N-monosubstitués, répondant aux formules générales I et II

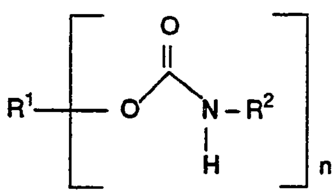

und

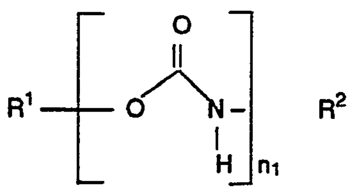

I II

dans lesquels

n est un nombre entier valant 1 à 6, avantageusement 1 à 4,

$n_1$ est un nombre entier valant 1 à 6, avantageusement 1 à 3,

$R^1$ représente un reste d'hydrocarbure aromatique ayant de préférence 6 à 18 atomes de carbone, un reste d'hydrocarbure aliphatique ayant de préférence 1 à 18 atomes de carbone, un reste d'hydrocarbure cycloaliphatique ayant de préférence 4 à 30 atomes de carbone ou un reste d'hydrocarbure araliphatique ayant de préférence 7 à 30 atomes de carbone,

$R^2$ représente un reste d'hydrocarbure aliphatique ayant de préférence 1 à 18 atomes de carbone, un reste d'hydrocarbure cycloaliphatique ayant de préférence 4 à 30 atomes de carbone ou un reste hydrocarbure araliphatique ayant de préférence 7 à 20 atomes de carbone, avec un agent d'alkylation répondant à la formule générale III

$$R^3X \qquad \text{III}$$

dans laquelle

$R^3$ représente un reste d'hydrocarbure aromatique ayant de préférence 6 à 18 atomes de carbone, un reste d'hydrocarbure aliphatique ayant de préférence 1 à 18 atomes de carbone, un reste d'hydrocarbure cycloaliphatique ayant de préférence 7 à 30 atomes de carbone, un reste d'hydrocarbure araliphatique ayant de préférence 7 à 39 atomes de carbone, et

X représente un atome d'halogène, avantageusement Cl ou Br, un groupe sulfate, sulfonate, phosphate ou phosphonate.

2. Procédé selon la revendication 1, caractérisé en ce qu'on conduit la réaction sans utiliser de solvant, en opérant dans un excès de l'agent d'alkylation.

3. Procédé selon la revendication 1, caractérisé en ce qu'on conduit la réaction dans un solvant organique aprotique, non polaire, en opérant éventuellement en présence de catalyseurs de transfert de phase.

4. Procédé selon la revendication 3, caractérisé en ce qu'on conduit la réaction dans du chlorobenzène, du diméthylformamide, de la N-méthylpyrrolidone et/ou de la tétraméthylènesulfone.

5. Procédé selon les revendications 1 à 3, caractérisé en ce qu'on conduit la réaction en présence d'hydroxydes de métaux alcalins, avantageusement d'hydroxyde de sodium.

6. Procédé selon les revendications 1 à 4, caractérisé en ce qu'on utilise comme agents d'alkylation le chlorure de méthyle, le bromure de méthyle, le chlorure d'éthyle, le bromure d'éthyle, le chlorure de dodécyle, le bromure de dodécyle, le chlorure d'allyle et le bromure d'allyle, le chlorure de benzyle ou un ester ptosylique.